# FASCICULE DE BREVET EUROPEEN

(11) **EP 2 873 437 B1**
(45) Date de publication et mention de la délivrance du brevet: **15.02.2017**
(21) Numéro de dépôt: 14187823.1
(22) Date de dépôt: 06.10.2014
(51) Int. Cl.: A61N 1/375

(54) **Traversée électrique pour boitier de dispositif médical actif**
Elektrische Kabeldurchführung für Gehäuse einer aktiven medizinischen Vorrichtung
Electric bushing for housing of active medical device

(30) Priorité: 13.11.2013 FR 1361056
(43) Date de publication de la demande: 20.05.2015
(73) Titulaire: Sorin CRM SAS, 92140 Clamart (FR)
(72) Inventeur: Boutaud, Bertrand, 75014 Paris (FR)
(74) Mandataire: Grünecker Patent- und Rechtsanwälte PartG mbB

(56) Documents cités:
- EP-A1- 2 377 573
- WO-A2-2008/067519
- US-A- 5 166 097
- US-A- 5 322 816

## Description

L'invention concerne la technologie de fabrication des "dispositifs médicaux implantables actifs" tels que définis par la directive 90/385/CEE du 20 juin 1990 du Conseil des communautés européennes.

Cette définition inclut notamment les appareils chargés de surveiller en continu l'activité cardiaque et délivrer si nécessaire au coeur des impulsions électriques de stimulation, de resynchronisation et/ou de défibrillation en cas de trouble du rythme détecté par le dispositif.

Elle inclut aussi les appareils neurologiques, les implants cochléaires, etc., ainsi que les dispositifs de mesure de pH ou autres paramètres intracorporels.

Ces dispositifs comportent un générateur constitué d'un boitier métallique, généralement en titane, sur lequel est montée une tête de connecteur. Le connecteur est pourvu de logements permettant de raccorder mécaniquement et électriquement au boitier du générateur une ou plusieurs sondes portant à leur extrémité distale diverses électrodes de recueil, de stimulation et/ou de défibrillation.

La liaison du connecteur aux circuits électroniques implique la réalisation de plusieurs traversées électriques, entre le connecteur monté sur la face supérieure du boitier ("côté externe") et le volume intérieur du boitier où se trouvent les circuits ("côté interne").

Outre les liaisons à la tête de connecteur, d'autres traversées peuvent être également prévues, par exemple pour assurer une liaison avec une électrode de surface disposée sur la face externe du boitier, ou encore au niveau d'un capteur intégré à une sonde du dispositif. Ces traversées peuvent être également rencontrées dans des sous-composants des dispositifs médicaux tels que les batteries et les condensateurs.

Une telle traversée est par exemple décrite dans le EP 2 377 573 A1 (Sorin CRM SAS).

La technique décrite dans ce document consiste, côté externe, à oxyder superficiellement le titane du boitier ou y déposer une couche isolante (par exemple de dioxyde de silicium) puis, côté interne, à creuser la paroi dans toute son épaisseur de manière à y former une gorge de contour fermé délimitant une zone ou "ilot" dédié à la conduction électrique. Cet ilot, créé dans la masse de la paroi du boitier, est isolé physiquement et donc électriquement du reste du corps du boitier.

Une liaison électrique est ensuite réalisée de chaque côté de l'ilot en ménageant sur chaque face de celui-ci une plage de contact sur laquelle est par exemple soudé un fil de liaison à une borne du circuit électronique, ou un picot de liaison du connecteur.

La présence côté externe de la couche externe isolante, qui n'est pas creusée, assure une herméticité parfaite de la traversée et évite toute pénétration de fluide à l'intérieur du boitier. Cette couche présente également l'avantage d'être biocompatible, biostable et résistante à la corrosion.

Le procédé peut être par ailleurs aisément industrialisé, sans surcoût important, dans la mesure où il ne fait appel qu'à des techniques conventionnelles éprouvées.

En revanche, cette technique laisse subsister une certaine fragilité mécanique.

En effet, après creusement de la gorge périphérique entourant l'ilot, ce dernier est entièrement détaché du reste de la paroi du boitier (ce qui est indispensable pour, précisément, assurer l'isolement électrique de la traversée) et n'est plus relié à cette paroi que par la mince couche superficielle qui forme une "membrane" ou un "diaphragme" d'oxyde dont l'épaisseur est typiquement de 10 à 15 µm (pour une épaisseur de paroi de l'ordre de 300 µm).

Cette fragilité résiduelle, intrinsèque à la minceur de la couche d'oxyde, est encore accrue par les tolérances assez moyennes obtenues en fond de trou lors du creusement de la gorge, pouvant conduire localement à l'apparition de fissures ou autres microdéfauts, précisément à proximité de la fine couche d'oxyde qui retient l'ilot et là où peuvent se concentrer des contraintes sollicitant la structure, par exemple du fait des fils ou picots soudés sur l'ilot central.

Un premier but de l'invention est de résoudre cette difficulté, en proposant une solution de renforcement mécanique de cette structure connue pour la rendre plus robuste et plus tolérante à la présence éventuelle de microdéfauts.

Un autre inconvénient inhérent à la structure de traversée connue décrite ci-dessus est qu'elle ne permet que de véhiculer passivement (conduction ohmique, purement résistive) un signal électrique entre l'intérieur et l'extérieur du boitier.

De ce fait, si l'on veut prévoir un filtrage (série ou parallèle) de la traversée, il est nécessaire de prévoir un disque capacitif externe reporté sur la face interne de la traversée, à laquelle ce disque sera relié mécaniquement et électriquement par exemple par un collage conducteur. Il s'agit d'une étape relativement coûteuse du point de vue industriel car elle nécessite de nombreuses sous-étapes qui par ailleurs peuvent engendrer des problèmes de fiabilité.

Un autre but encore de l'invention est de procurer divers avantages collatéraux, notamment la faculté d'intégrer à la structure connue, perfectionnée selon l'invention, des éléments fonctionnels additionnels tels qu'antenne RF (pour des fonctions de télémétrie RF), électrodes, éléments de capteur, etc.

En effet, si l'on prend l'exemple des antennes RF, celles-ci sont actuellement réalisées à partir d'un fil métallique extérieur au boitier, fil qui est connecté aux circuits électroniques intérieurs via une traversée dédiée et qui est ensuite surmoulé dans une matrice plastique biocompatible. Ici encore, la connectique et l'industrialisation sont complexes, et induisent un volume hors tout du dispositif significativement plus élevé que celui des appareils dépourvus d'antenne RF.

Ces buts, ainsi que d'autres, sont atteints par l'invention, qui a pour objet un boitier de dispositif médical actif du type général décrit dans le EP 2 377 573 A1 précité, c'est-à-dire un boitier (ou élément de boitier) comprenant une paroi métallique présentant un côté externe et un côté interne, cette paroi étant pourvue d'au moins une traversée hermétique et électriquement isolée pour une liaison électrique au travers de la paroi. Le boitier comportant en outre dans la région de la traversée : côté interne de la paroi, une gorge de contour fermé délimitant dans la paroi un ilot métallique physiquement et électriquement isolé du reste de la paroi, cette gorge s'étendant dans toute l'épaisseur de la paroi ; et côté externe de la paroi, une couche externe électriquement isolante formée au-dessus de la paroi et s'étendant sur une région située au moins au droit de la gorge et au-delà de part et d'autre de la gorge, cette couche externe isolante comprenant un évidement formé au droit de l'ilot dans toute l'épaisseur de la couche externe isolante.

De façon caractéristique de l'invention, ce boitier comprend en outre, dans la région de la traversée côté externe de la paroi, une couche externe électriquement conductrice formée au-dessus de la couche externe isolante et s'étendant sur ladite région située au moins au droit de la gorge et au-delà de part et d'autre de la gorge, l'ilot étant supporté mécaniquement et de manière étanche par les deux couches externe isolante et externe conductrice.

La couche externe électriquement conductrice peut être une couche métallique de titane, de platine, de palladium, d'or ou d'alliages de ceux-ci. La couche externe isolante peut être une couche d'oxyde du métal de la paroi, formée sur une fraction de l'épaisseur de cette paroi, ou bien une couche rapportée, déposée en surface de la paroi.

Selon un premier mode de réalisation de l'invention, l'évidement s'étend également dans toute l'épaisseur de la couche externe conductrice, de manière à ménager en fond d'évidement une plage de contact à l'ilot côté extérieur du boitier dans la région de la traversée.

Dans ce cas, la couche externe conductrice peut s'étendre au-delà de la région de la traversée sur une étendue définissant une première armature d'un condensateur de filtrage parallèle de la traversée, l'autre armature de ce condensateur étant formée par la région de la paroi s'étendant en regard de la première armature.

Selon un second mode de réalisation de l'invention, l'évidement est rempli du matériau de la couche externe conductrice, de manière à relier électriquement l'ilot à la couche externe conductrice dans la région de la traversée.

Dans ce cas, le boitier peut comporter en outre dans la région de la traversée, côté interne de la paroi : une couche interne électriquement isolante formée au-dessus de la paroi et s'étendant sur la région de l'ilot ; et une couche interne électriquement conductrice formée au-dessus de la couche interne isolante et s'étendant sur la région de l'ilot, de manière à former une plage de contact à l'ilot côté intérieur du boitier. La couche interne conductrice définit ainsi une première armature d'un condensateur de filtrage série de la traversée, l'autre armature de ce condensateur étant formée par la région de la paroi s'étendant en regard de la première armature.

D'autre part, la couche externe conductrice peut s'étendre au-delà de la région de la traversée selon un motif prédéterminé définissant un élément d'antenne radiofréquence, d'électrode de capteur, ou d'électrode de détection/stimulation.

Enfin, le boitier peut comporter sur la région de la traversée, côté externe de la paroi, un empilement, formé dans la couche externe isolante, de couches externes additionnelles multiples alternativement conductrices et isolantes.

On va maintenant décrire un exemple de mise en oeuvre de l'invention, en référence aux dessins annexés où les mêmes références numériques désignent d'une figure à l'autre des éléments identiques ou fonctionnellement semblables.
La Figure 1 est une vue en élévation, en coupe, d'une traversée selon l'état de la technique.
La Figure 2 est une vue en élévation, en coupe, d'une traversée selon un premier mode de réalisation de l'invention.
La Figure 3 est une vue en élévation, en coupe, d'une traversée selon un second mode de réalisation de l'invention.
La Figure 4 est une vue de dessus montrant une antenne RF intégrée au boitier du dispositif actif et réalisée en appliquant les enseignements du second mode de réalisation de l'invention.
Les Figures 5 et 6 sont homologues des Figures 2 et 3, pour illustrer un perfectionnement dans lequel la traversée comporte en outre un condensateur série de filtrage.
La Figure 7 illustre un perfectionnement du second mode de réalisation de l'invention mettant en oeuvre une structure multicouche.
La Figure 8 illustre les étapes successives de réalisation de la structure de la Figure 2 selon le premier mode de réalisation de l'invention.
La Figure 9 illustre les étapes successives de réalisation de la structure de la Figure 3 selon le second mode de réalisation de l'invention.
La Figure 10 illustre une variante de préparation du substrat destiné à la réalisation de la traversée selon l'invention.

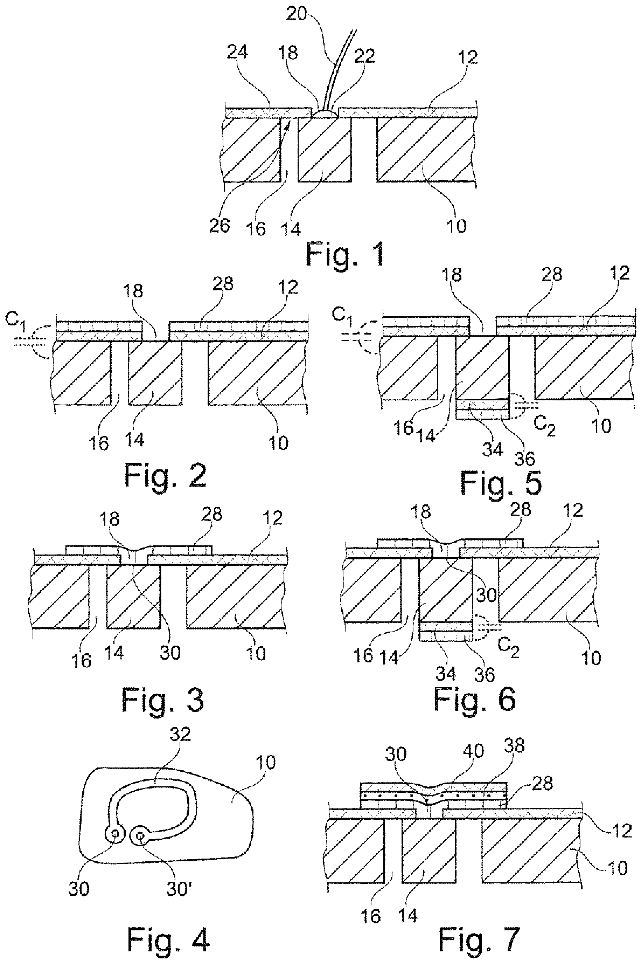

La Figure 1 représente une structure selon l'état de la technique, dont les détails de réalisation sont décrits dans le EP 2 377 573 A1 précité.

La référence 10 désigne le boitier métallique en titane du générateur, ou bien une platine de titane formant une pièce métallique distincte qui sera ensuite rapportée sur le boitier et soudée à celui-ci.

Une couche isolante 12 est formée au-dessus de la paroi 10, au moins sur la face externe du boitier. Cette couche isolante 12 peut être réalisée par oxydation du titane du boitier 10 sur une profondeur contrôlée, ou par dépôt d'une couche de matériau isolant, par exemple de dioxyde de silicium, en surface de l'épaisseur 10 du boitier en titane. L'épaisseur de la couche d'oxyde isolante 12 est par exemple de l'ordre de 10 µm pour une épaisseur de boitier de l'ordre de 300 µm.
La structure comporte en outre un ilot conducteur 14 ménagé dans l'épaisseur du boitier 10 par creusement d'une gorge 16 :
- dans le sens de la profondeur, la gorge 16 est creusée depuis le côté interne du boitier dans toute l'épaisseur de la paroi 10 du boitier métallique. En revanche, la couche isolante 12 est laissée intacte, de manière que l'ilot 14 puisse être supporté par le pont de matière formé par cette couche isolante 12 entre la zone de l'ilot et le reste de la couche métallique du boitier. La couche externe isolante 12 forme en outre une barrière hermétique entre l'intérieur du boitier et l'environnement extérieur ;
- dans le plan de la surface du boitier, la gorge 16 est creusée sur un contour fermé, de manière à complètement isoler physiquement et donc électriquement l'ilot 14 du reste du boitier 10 sur toute sa périphérie.

Cette structure comporte également une ouverture 18 formée côté externe dans l'épaisseur de la couche d'oxyde isolante 12 au droit de l'ilot 14, de manière à mettre à nu une zone sur laquelle pourra être brasé un fil 20 ou un picot pour assurer la prise de contact électrique, via la brasure 22, avec l'ilot conducteur central 14.

Côté interne, l'ilot 14 sera relié à un fil de liaison aux circuits électroniques enfermés dans le boitier 10 du générateur par exemple par brasure d'un fil de liaison (non représenté), de manière à réaliser une traversée étanche et isolée du boitier, depuis ce fil de liaison interne jusqu'au picot ou fil extérieur 20, du côté opposé du boitier 10. D'autres techniques de connexion peuvent être employées en variante, telles que soudure, *wire bonding* ou contact par l'intermédiaire d'un élément élastique conducteur.

Cette structure connue présente la caractéristique que l'ilot 14 n'est relié, et mécaniquement supporté, que par le mince pont de matière 24 de la couche d'oxyde 12.

Cette région, notamment le fond 26 de la gorge 16, est particulièrement fragile et il peut apparaitre au cours du procédé de fabrication des défauts ou des microfissures susceptibles d'affaiblir le pont ou "diaphragme" 24, déjà fragile du fait de sa très faible épaisseur.

La Figure 2 illustre un premier mode de réalisation de l'invention permettant de remédier à ce défaut, et de procurer en outre des avantages collatéraux que l'on exposera plus bas.

Le principe de l'invention consiste à déposer au-dessus de la couche externe isolante d'oxyde 12 une couche externe supplémentaire 28, conductrice, par exemple par dépôt d'une métallisation de titane ou d'un autre matériau tel que platine, palladium, or et leurs alliages sur une épaisseur de l'ordre de quelques centaines de nanomètres à quelques micromètres. Le titane est toutefois préféré du fait de sa meilleure affinité avec l'épaisseur de la paroi 10 sous-jacente (même coefficient de dilatation) et de ses propriétés bien connues de biocompatibilité.

Dans le mode de réalisation illustré Figure 2, la couche externe conductrice 28 est déposée avant creusement de l'ouverture 18 destinée à réaliser la prise de contact avec l'ilot conducteur 14, ce qui permet de réduire les contraintes de manipulation sur la structure, en renforçant celle-ci en particulier lors du creusement de la gorge 16. Le processus comprend donc la formation de la couche externe isolante 12 (dépôt d'un matériau rapporté ou oxydation superficielle du titane de la paroi 10), puis de la couche externe conductrice 28 (métallisation) sur toute l'étendue de la face externe de la paroi 10.

L'ouverture 18 est réalisée dans une étape ultérieure, de manière à mettre à nu une plage de contact avec l'ilot conducteur central 14 en vue de la brasure d'un fil ou d'un picot, comme dans la configuration illustrée Figure 1.

En même temps que le creusement de l'ouverture de prise de contact 18, il est possible de graver le contour de la métallisation 28 et de la couche externe isolante 12 sur une surface prédéterminée, de manière à définir une structure de condensateur C₁, les couches 10 et 28 formant les armatures de ce condensateur et la couche isolante d'oxyde 12 en formant le diélectrique. Si l'on relie à la masse la couche externe conductrice 28, on dispose ainsi d'un élément de filtrage parallèle intégré à la traversée électrique du boitier. La couche isolante d'oxyde 12 est structurée de la manière voulue, par exemple par photolithographie.

La structure ainsi obtenue présente les avantages suivants :
- renforcement de la rigidité mécanique et de l'étanchéité du pont de matière reliant mécaniquement l'ilot 14 au reste de la paroi du boitier 10 (du fait de l'augmentation de l'épaisseur de matière par ajout de la couche externe 28), ainsi que de l'étanchéité ;
- intégration d'un élément capacitif de filtrage (d'une valeur typique de l'ordre de 500 pF), sans report d'aucun composant supplémentaire ;
- possibilité de structurer la couche externe conductrice 28 et la couche externe isolante 12 de manière à réaliser des éléments tels qu'une électrode de détection, un élément de capteur capacitif ou de détecteur biochimique, etc.

La Figure 3 illustre un second mode de réalisation de l'invention.

Par rapport au précédent, dans ce mode de réalisation la couche isolante d'oxyde 12 est structurée (notamment pour dégager la plage de contact avec l'ouverture 18) avant dépôt de la couche externe conductrice (métallisation) 28.

De ce fait, lorsque le matériau de la couche conductrice 28 est déposé au cours de l'étape de métallisation, ce matériau pénètre dans l'ouverture 18 formée dans la couche d'oxyde 12 et vient en contact en 30 avec l'ilot conducteur 14, assurant ainsi une continuité électrique entre d'une part cet ilot 14 et les circuits auxquels il est relié côté intérieur, et d'autre part la couche externe conductrice 28.

Outre le renforcement de la rigidité mécanique et de l'étanchéité du pont de matière reliant l'ilot au reste du boitier (de la même manière que dans le premier mode de réalisation illustré Figure 2), cette technique permet d'élargir la connectivité électrique avec l'extérieur, grâce au contact direct entre la métallisation externe (couche conductrice 28) et l'ilot central 14. Un premier avantage de cette variante de réalisation est la possibilité de déporter la connectique extérieure ailleurs qu'à la verticale de l'ilot, évitant ainsi de solliciter mécaniquement la fragile zone microstructurée de façon directe par un fil ou un picot assurant la prise de contact électrique juste à cet endroit.

Un autre avantage est la possibilité de graver la couche externe conductrice 28 de manière à définir des éléments électroniques qui seront électriquement reliés à l'ilot, par exemple comme illustré Figure 4 une antenne RF en forme de boucle 32 s'étendant selon une géométrie parfaitement contrôlée entre deux points de contact 30 et 30' à des ilots conducteurs respectifs reliés, côté intérieur, aux circuits électroniques du générateur. Une telle antenne pourra notamment être utilisée à des fins de communication sans fil (télémétrie) et/ou de recharge d'une batterie via un couplage inductif. La technique de l'invention permet de lui donner aisément toute forme appropriée telle que boucle, spirale, carré, etc. ou même simple antenne rectiligne.

Il est également possible de structurer la couche externe conductrice 28 de manière à définir une électrode de détection/stimulation ou une surface destinée à un capteur d'autres variables physicochimiques ou paramètres physiques détectables par des variations d'impédance ohmique et/ou capacitive, l'électrode métallique assurant une surface de captage supérieure et indépendante du micro-usinage dans le titane. D'autre part, la métallisation externe peut aussi servir à une détection de type capacitif, grâce à la structure de condensateur définie par les couches conductrices 10 et 28 séparées par la couche isolante 12.

Les Figures 5 et 6 sont homologues des Figures 2 et 3, pour un perfectionnement consistant à former un filtre capacitif série directement intégré à la traversée. Ce perfectionnement est applicable à l'un ou l'autre des modes de réalisation décrits plus haut.

Pour réaliser ce filtre série, la paroi 10 du boitier comporte, côté interne, une couche isolante 34 revêtue d'une couche conductrice 36, ces couches internes 34, 36 étant réalisées de la même manière que les couches externes correspondantes 12, 28, par mise en oeuvre de techniques comparables, ces couches 34, 36 pouvant notamment être déposées sur la face interne de la paroi 10 avant creusement de la gorge 16.

Dans le plan de la surface du boitier, ces couches 34, 36 s'étendent sur la surface de l'ilot 14 et définissent un condensateur C₂ dont les armatures sont formées par le titane de la paroi 10 dans la région de l'ilot et par la couche interne conductrice 36, d'une part, et le diélectrique est formé par la couche interne isolante 34, d'autre part. Si l'on soude sur la couche interne conductrice 36 un fil de liaison vers les circuits internes du générateur, on réalise ainsi une traversée intégrant un filtre capacitif série C₂, sans report d'aucun composant additionnel, par exemple à des fins de filtrage complémentaire.

La Figure 7 illustre un autre perfectionnement, applicable au mode de réalisation de la Figure 3.

Dans ce cas, au-dessus de la couche externe conductrice 28 sont formées des couches supplémentaires 38, 40... alternativement isolantes ou conductrices, de manière à définir côté externe du boitier un empilement multiple. L'empilement de ces couches multiples présente le double intérêt de :
- pouvoir constituer un élément tel qu'un capteur ou bien une fonction électronique avancée nécessitant plusieurs couches (transistor par exemple) ; et
- renforcer encore la structure du point de vue mécanique et de l'étanchéité, les couches successivement déposées augmentant l'épaisseur du pont de matière reliant l'ilot conducteur 14 au reste du boitier.

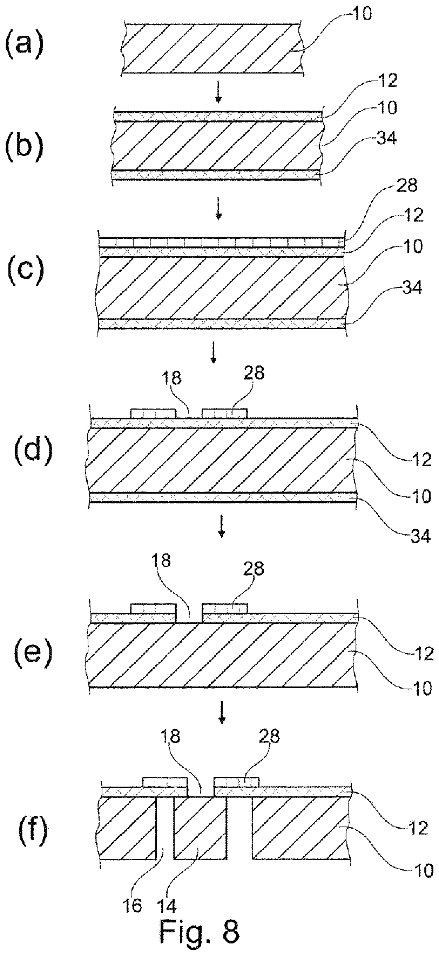

La Figure 8 illustre les étapes successives de réalisation de la structure de la Figure 2 selon le premier mode de réalisation de l'invention.

L'élément de départ est le boitier en titane 10 (étape a), sur lequel sont créées de part et d'autre des couches isolantes, avec une couche isolante externe 12 et une couche isolante externe 34 (étape b). Ces couches peuvent être réalisées par oxydation du titane du boitier 10 sur une profondeur contrôlée, ou par dépôt d'une couche de matériau isolant, par exemple de dioxyde de silicium en surface de l'épaisseur du boitier 10. L'épaisseur de chacune des couches 12, 34, est par exemple de l'ordre de 10 µm pour une épaisseur de boitier de l'ordre de 300 µm.

Ces couches 12 et 34 peuvent être réalisées par exemple par oxydation thermique, ou par tout autre procédé tel qu'oxydation plasma ou dépôt chimique. Il est également possible de procéder par anodisation, en soumettant le boitier à une différence de potentiel et en le mettant simultanément en contact avec une solution d'eau et d'acide sulfurique, par trempage ou bien à l'aide d'un pinceau-électrode.

L'étape suivante (étape c) consiste à former côté externe la couche conductrice 28, par exemple par dépôt sous vide.

L'étape suivante (étape d) est une étape de structuration de la couche externe conductrice 28, notamment pour délimiter l'ouverture 18 qui permettra ultérieurement de réaliser la prise de contact, et pour délimiter l'étendue de cette couche métallique 28 afin notamment d'ajuster la valeur du condensateur série C₁ (Figure 2) associé à la traversée.

L'étape suivante (étape e) est une étape de gravure de la couche externe isolante d'oxyde 12, notamment pour réaliser la mise à nu de la couche 10 du boitier conductrice côté externe par l'ouverture 18. Cette étape peut également s'accompagner d'une suppression, facultative, de la couche d'oxyde isolante 34 du côté intérieur du boitier.

L'étape suivante (étape f) consiste à ménager l'ilot conducteur 14 physiquement isolé dans l'épaisseur du boitier 10 par creusement de la gorge 16 dans toute l'épaisseur de ce boitier. Le creusement de cette gorge peut être réalisé par divers procédés en eux-mêmes connus, par exemple par gravure sélective chimique (interactions d'espèces réactives avec le titane) ou physique (bombardement ionique), ou encore par tout procédé de microstructuration, gravure laser, etc., ces procédés pouvant être également combinés entre eux pour minimiser le temps de découpe du titane. On notera par ailleurs que la gorge n'est pas nécessairement cylindrique, elle peut étalement présenter une forme conique, par exemple si l'on recourt à une technique de gravure humide qui n'est par parfaitement directionnelle.

La structure finalement obtenue à l'issue de cette étape f est celle illustrée par ailleurs à la Figure 2.

On notera que dans cette mise en oeuvre l'étape de formation de l'ilot (étape f) est exécutée après les étapes de dépôt de la métallisation (étape c) et de structuration de la couche d'oxyde isolante extérieure (étape e). Ainsi, les contraintes éventuellement subies par le matériau ne risquent pas de fragiliser la structure finale, dans la mesure où la paroi 10 du boitier est encore massive, n'ayant pas encore été entamée par le creusement de la gorge 16.

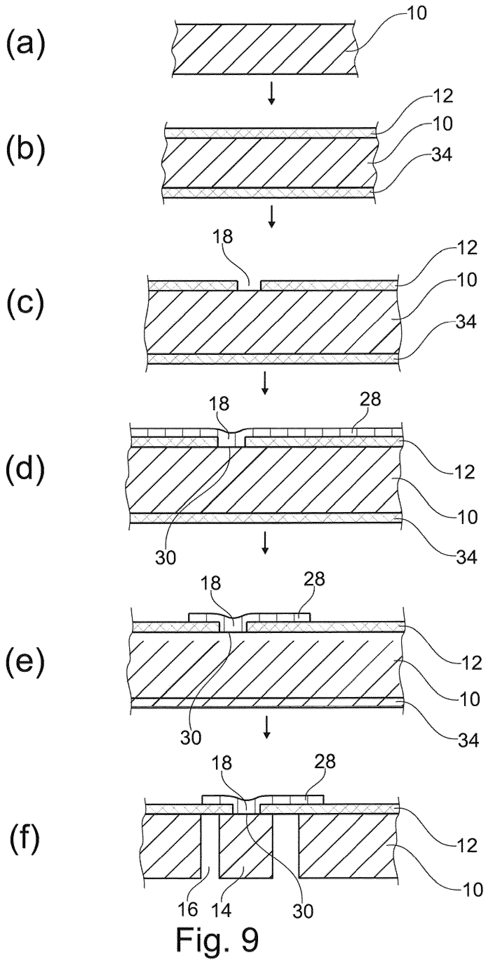

La Figure 9 illustre les étapes successives de réalisation de la structure de la Figure 3 selon le second mode de réalisation de l'invention.

Les premières étapes a et b sont identiques à celles de la Figure 8 du mode de réalisation précédent.

En revanche, l'étape qui suit (étape c) est une étape de structuration de la couche externe isolante d'oxyde 12, selon des techniques comparables à celles qui ont été évoquées à propos de l'étape e de la Figure 7, notamment pour former l'ouverture 18.

L'étape suivante (étape d) est celle du dépôt de la couche externe conductrice 28 par métallisation, selon des techniques comparables à celles qui ont été exposées plus haut au sujet de l'étape c de la Figure 8, à la seule différence que ce dépôt intervient après structuration de la couche d'oxyde sous-jacente 12 - ce qui permet notamment de remplir l'ouverture 18, formée à l'étape précédente, de manière à réaliser la prise de contact direct 30 avec le titane de la paroi 10 du boitier.

L'étape suivante (étape e) est une structuration de la couche externe conductrice 28, selon des techniques comparables à ce qui a été exposé plus haut à propos de l'étape d de la Figure 8. Cette étape peut être suivie d'une étape, facultative, d'élimination de la couche interne isolante d'oxyde 34 sur toute l'étendue du substrat.

L'étape finale (étape f) est la formation de l'ilot 14 par creusement de la gorge 16, de la même manière qu'à l'étape correspondante f de la Figure 8. La structure finale obtenue à l'issue de cette étape f est celle illustrée Figure 3.

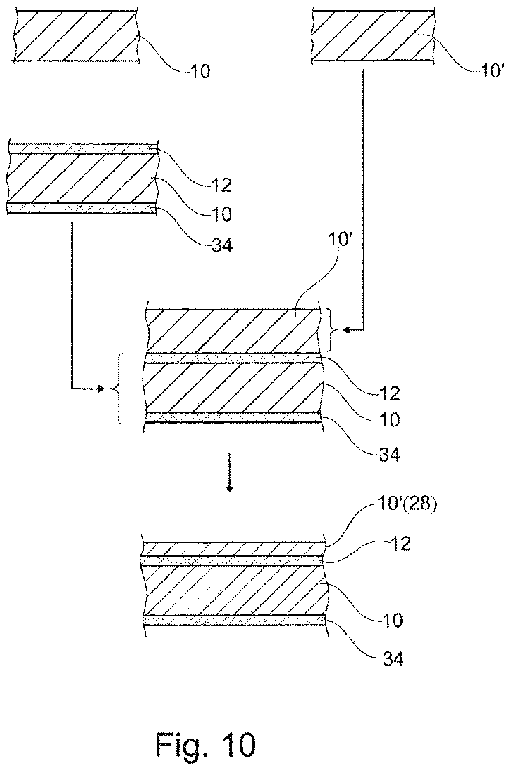

La Figure 10 illustre une variante de préparation du substrat destiné à la réalisation de la traversée selon l'invention.

Dans cette variante, au lieu de partir d'un substrat homogène de titane sur lequel est déposée une couche de métallisation, on prépare séparément deux substrats en titane 10 et 10' d'épaisseur identique (typiquement 300 µm), ou non, et l'on forme sur l'un d'eux (comme illustré), ou sur les deux, une couche d'oxyde 12, 34 de la même manière que ce qui a été décrit plus haut pour l'étape b illustrée Figure 8.

Les deux substrats sont ensuite superposés et soudés l'un à l'autre par un procédé thermique, de thermo-compression ou autre. Pour faciliter cette étape de soudage, une couche additionnelle peut être ajoutée au préalable à l'un ou l'autre des substrats 10 ou 10'.

On obtient à l'issue de cette opération un substrat composite incluant une couche interne d'oxyde 12 (qui à l'origine était présente sur l'un et/ou l'autre des substrats 10, 10' avant leur soudage) et supportant une couche conductrice externe, à savoir l'épaisseur du substrat de titane 10'. Après amincissement, cette couche 10' pourra jouer le même rôle que la métallisation 28 des modes de réalisation précédents, la structure de base obtenue étant comparable à celle obtenue précédemment à l'issue de l'étape c illustrée Figures 8.

Les étapes suivantes de procédé sont similaires à ce qui a été décrit plus haut pour les étapes d à f de la Figure 8, en partant de cette structure de base composite.

## Revendications

1. Un boitier ou élément de boitier de dispositif électronique médical actif, comprenant une paroi métallique (10) présentant un côté externe et un côté interne, cette paroi étant pourvue d'au moins une traversée hermétique et électriquement isolée pour une liaison électrique au travers de la paroi,
le boitier ou élément de boitier comportant en outre dans la région de la traversée :
- côté interne de la paroi, une gorge de contour fermé (16) délimitant dans la paroi un ilot métallique (14) physiquement et électriquement isolé du reste de la paroi, cette gorge s'étendant dans toute l'épaisseur de la paroi ; et
- côté externe de la paroi, une couche externe électriquement isolante (12) formée au-dessus de la paroi et s'étendant sur une région située au moins au droit de la gorge et au-delà de part et d'autre de la gorge, cette couche externe isolante comprenant un évidement (18) formé au droit de l'ilot dans toute l'épaisseur de la couche externe isolante,
boitier ou élément de boitier **caractérisé en ce qu'**il comporte en outre, dans la région de la traversée :
- côté externe de la paroi, une couche externe électriquement conductrice (28) formée au-dessus de la couche externe isolante et s'étendant sur ladite région située au moins au droit de la gorge et au-delà de part et d'autre de la gorge,
l'ilot étant supporté mécaniquement et de manière étanche par les deux couches externe isolante et externe conductrice.

2. Le boitier ou élément de boitier de la revendication 1, dans lequel l'évidement s'étend également dans toute l'épaisseur de la couche externe conductrice, de manière à ménager en fond d'évidement une plage de contact à l'ilot côté extérieur du boitier ou élément de boitier dans la région de la traversée.

3. Le boitier ou élément de boitier de la revendication 1, dans lequel l'évidement est rempli (30) du matériau de la couche externe conductrice, de manière à relier électriquement l'ilot à la couche externe conductrice dans la région de la traversée.

4. Le boitier ou élément de boitier de la revendication 1, dans lequel la couche externe conductrice s'étend au-delà de la région de la traversée sur une étendue définissant une première armature d'un condensateur (C₁) de filtrage parallèle de la traversée, l'autre armature de ce condensateur étant formée par la région de la paroi s'étendant en regard de la première armature.

5. Le boitier ou élément de boitier de la revendication 1, dans lequel le boitier ou élément de boitier comporte en outre dans la région de la traversée, côté interne de la paroi :
- une couche interne électriquement isolante (34) formée au-dessus de la paroi et s'étendant sur la région de l'ilot ; et
- une couche interne électriquement conductrice (36) formée au-dessus de la couche interne isolante et s'étendant sur la région de l'ilot, de manière à former une plage de contact à l'ilot côté intérieur du boitier ou élément de boitier,
la couche interne conductrice définissant une première armature d'un condensateur (C₂) de filtrage série de la traversée, l'autre armature de ce condensateur étant formée par la région de la paroi s'étendant en regard de la première armature.

6. Le boitier ou élément de boitier de la revendication 3, dans lequel la couche externe conductrice s'étend au-delà de la région de la traversée selon un motif prédéterminé définissant un élément d'antenne radiofréquence (32), d'électrode de capteur, ou d'électrode de détection/stimulation.

7. Le boitier ou élément de boitier de la revendication 3, dans lequel le boitier ou élément de boitier comporte en outre sur la région de la traversée, côté externe de la paroi, un empilement, formé dans la couche externe isolante, de couches externes additionnelles multiples (38, 40) alternativement conductrices et isolantes.

8. Le boitier ou élément de boitier de la revendication 1, dans lequel la couche externe électriquement conductrice est une couche métallique de titane, de platine, de palladium, d'or ou d'alliages de ceux-ci.

9. Le boitier ou élément de boitier de la revendication 1, dans lequel la couche externe isolante est une couche d'oxyde du métal de la paroi, formée dans une fraction de l'épaisseur de cette paroi.

10. Le boitier ou élément de boitier de la revendication 1, dans lequel la couche externe isolante est une couche rapportée, déposée en surface de la paroi.

## Patentansprüche

1. Gehäuse oder Gehäuseelement einer aktiven medizinischen Elektronikvorrichtung, umfassend eine Metallwand (10), die eine Außenseite und eine Innenseite aufweist, wobei diese Wand mit mindestens einer hermetischen und elektrisch isolierten Durchführung für eine elektrische Verbindung durch die Wand hindurch aufweist,
wobei das Gehäuse oder Gehäuseelement in der Region der Durchführung ferner Folgendes aufweist:
- auf der Innenseite der Wand eine Rille mit geschlossener Kontur (16), die in der Wand eine metallische Insel (14) begrenzt, die von der übrigen Wand physikalisch und elektrisch isoliert ist, wobei diese Rille sich in der gesamten Dicke der Wand erstreckt; und
- auf der Außenseite der Wand eine elektrisch isolierende äußere Schicht (12), die oberhalb der Wand gebildet ist und sich über eine Region erstreckt, die sich mindestens rechtwinklig zu der Rille und zu beiden Seiten über die Rille hinaus befindet, wobei diese isolierende äußere Schicht eine Ausnehmung (18) umfasst, die rechtwinklig zu der Insel in der gesamten Dicke der isolierenden äußeren Schicht gebildet ist,
wobei das Gehäuse oder Gehäuseelement **dadurch gekennzeichnet ist, dass** es in der Region der Durchführung ferner Folgendes aufweist:
- auf der Außenseite der Wand eine elektrisch leitende äußere Schicht (28), die oberhalb der isolierenden äußeren Schicht gebildet ist und sich über die Region erstreckt, die sich mindestens rechtwinklig zu der Rille und zu beiden Seiten über die Rille hinaus befindet,
wobei die Insel durch die zwei Schichten, die isolierende und die leitende äußere Schicht, mechanisch und abdichtend gestützt wird.

2. Gehäuse oder Gehäuseelement nach Anspruch 1, wobei die Ausnehmung sich auch in der gesamten Dicke der äußeren leitenden Schicht in der Form erstreckt, dass sie am Boden der Ausnehmung einen Kontaktbereich mit der Insel auf der Außenseite des Gehäuses oder Gehäuseelements in der Region der Durchführung ausbildet.

3. Gehäuse oder Gehäuseelement nach Anspruch 1, wobei die Ausnehmung mit dem Material der äußeren leitenden Schicht in der Form gefüllt (30) ist, dass sie die Insel mit der äußeren leitenden Schicht in der Region der Durchführung elektrisch verbindet.

4. Gehäuse oder Gehäuseelement nach Anspruch 1, wobei die leitende äußere Schicht sich über die Region der Durchführung hinaus über eine Ausdehnung erstreckt, die eine erste Armatur eines Filterkondensators (C₁) parallel zu der Durchführung definiert, wobei die andere Armatur dieses Kondensators durch die Region der Wand gebildet ist, die sich der ersten Armatur gegenüber erstreckt.

5. Gehäuse oder Gehäuseelement nach Anspruch 1, wobei das Gehäuse oder Gehäuseelement in der Region der Durchführung auf der Innenseite der Wand ferner Folgendes aufweist:
- eine elektrisch isolierende innere Schicht (34), die oberhalb der Wand gebildet ist und sich über die Region der Insel erstreckt; und
- eine elektrisch leitende innere Schicht (36), die oberhalb der isolierenden inneren Schicht gebildet ist und sich über die Region der Insel in der Form erstreckt, dass sie einen Kontaktbereich mit der Insel auf der Innenseite des Gehäuses oder Gehäuseelements bildet,
wobei die leitende innere Schicht eine erste Armatur eines Filterkondensators (C₂) in Reihe mit der Durchführung definiert, wobei die andere Armatur dieses Kondensators durch die Region der Wand gebildet ist, die sich der ersten Armatur gegenüber erstreckt.

6. Gehäuse oder Gehäuseelement nach Anspruch 3, wobei sich die leitende äußere Schicht über die Region der Durchführung gemäß einem vorbestimmten Muster erstreckt, das ein Funkfrequenzantennen-Element (32), Sensorelektroden- oder Detektions-/Stimulationselektroden-Element definiert.

7. Gehäuse oder Gehäuseelement nach Anspruch 3, wobei das Gehäuse oder Gehäuseelement in der Region der Durchführung auf der Außenseite der Wand ferner einen Stapel aufweist, der in der isolierenden äußeren Schicht aus mehreren zusätzlichen äußeren Schichten (38, 40) gebildet ist, die abwechselnd leitend und isolierend sind.

8. Gehäuse oder Gehäuseelement nach Anspruch 1, wobei die elektrisch leitende äußere Schicht eine metallische Schicht aus Titan, aus Platin, aus Palladium, aus Gold oder aus Legierungen von diesen ist.

9. Gehäuse oder Gehäuseelement nach Anspruch 1, wobei die isolierende äußere Schicht eine Oxidschicht aus dem Metall der Wand ist, die in einem Bruchteil der Dicke dieser Wand gebildet ist.

10. Gehäuse oder Gehäuseelement nach Anspruch 1, wobei die isolierende äußere Schicht eine aufgebrachte Schicht ist, die an der Oberfläche der Wand abgeschieden ist.

## Claims

1. A case or case element for an active medical electronic device, comprising a metal wall (10) having an outer side and an inner side, such wall being provided with at least one hermetic and electrically insulated feedthrough for an electric connection through the wall,
the case or case element further including, in the feedthrough region:
- on the inner side of the wall, a closed-contour groove (16) delimiting in the wall a metal island (14) physically and electrically insulated from the remaining of the wall, this groove extending through the whole thickness of the wall; and
- on the outer side of the wall, an electrically insulating external layer (12) formed above the wall and extending over a region located at least at the groove and beyond on either side of the groove,
this insulating external layer comprising a cutout (18) formed at the island through the whole thickness of the insulating external layer,
said case or case element being **characterized in that** it further includes, in the feedthrough region:
- on the outer side of the wall, an electrically conductive external layer (28) formed above the insulating external layer and extending over said region located at least at the groove and beyond on either side of the groove,
the island being mechanically and hermetically supported by the two insulating external and conductive external layers.

2. The case or case element of claim 1, wherein the cutout also extends through the whole thickness of the conductive external layer, so as to form at the cutout bottom an area of contact with the outer side island of the case or case element in the feedthrough region.

3. The case or case element of claim 1, wherein the cutout is filled (30) with the material of the conductive external layer, so as to connect electrically the island to the conductive external layer in the feedthrough region.

4. The case or case element of claim 1, wherein the conductive external layer extends beyond the feedthrough region over an extent defining a first armature of a capacitor (C₁) for parallel filtering of the feedthrough, the other armature of this capacitor being formed by the region of the wall extending opposite the first armature.

5. The case or case element of claim 1, wherein the case or case element further includes in the feedthrough region, on the inner side of the wall:
- an electrically insulating internal layer (34) formed above the wall and extending over the island region; and
- an electrically conductive internal layer (36) formed above the insulating internal layer and extending over the island region, so as to form an area of contact with the inner side island of the case or case element,
the conductive internal layer defining a first armature of a capacitor (C₂) for series filtering of the feedthrough, the other armature of this capacitor being formed by the region of the wall extending opposite the first armature.

6. The case or case element of claim 3, wherein the conductive external layer extends beyond the feedthrough region according to a predetermined pattern defining a radiofrequency antenna (32), sensor electrode or detection/stimulation electrode element.

7. The case or case element of claim 3, wherein the case or case element further includes on the feedthrough region, on the outer side of the wall, a stack, formed in the insulating external layer, of multiple additional external layers (38, 40) alternately conductive and insulating.

8. The case or case element of claim 1, wherein the electrically conductive external layer is a metal layer made of titanium, platinum, palladium, gold or alloys thereof.

9. The case or case element of claim 1, wherein the insulating external layer is a layer of oxide of the wall metal, formed through a fraction of the thickness of this wall.

10. The case or case element of claim 1, wherein the insulating external layer is an added layer, deposited at the surface of the wall.
